Europäisches Patentamt

European Patent Office

Office européen des brevets

(19)

(11) Veröffentlichungsnummer: **0 321 924 B1**

(12) **EUROPÄISCHE PATENTSCHRIFT**

(45) Veröffentlichungstag der Patentschrift: **22.07.92**

(51) Int. Cl.5: **A61L 33/00**, A61B 5/00

(21) Anmeldenummer: **88121301.1**

(22) Anmeldetag: **20.12.88**

(54) **Polymermaterial, das bei Kontakt mit einem Proteine enthaltenden Material eine stabile Proteinschicht an der Oberfläche anlagert, bzw. Verwendung des Polymermateriales.**

(30) Priorität: **23.12.87 CH 5030/87**

(43) Veröffentlichungstag der Anmeldung:
**28.06.89 Patentblatt 89/26**

(45) Bekanntmachung des Hinweises auf die Patenterteilung:
**22.07.92 Patentblatt 92/30**

(84) Benannte Vertragsstaaten:
**AT BE CH DE ES FR GB IT LI LU NL SE**

(56) Entgegenhaltungen:
EP-A- 0 046 601    EP-A- 0 061 312
EP-A- 0 068 509    EP-A- 0 093 489
EP-A- 0 093 927    EP-A- 0 201 378
WO-A-84/01892    DE-A- 2 833 356
FR-A- 2 248 855    FR-A- 2 409 743
GB-A- 2 166 977

EXTENDED ABSTRACTS, Band 86, Nr. 2,
19-24. Oktober 1986, Seite 875, Zusammenfassung Nr. 583; Princeton, N.J., US:
"Development of single-fiber optical sensors
for blood gas measurements"

(73) Patentinhaber: **Willi Möller AG**
**Gubelstrasse 37**
**CH-8050 Zürich(CH)**

(72) Erfinder: **Simon, Wilhelm**
**Hadlaubstrasse 63**
**CH-8006 Zürich(CH)**
Erfinder: **Dürselen, Lucas F.J.**
**Saizig 7**
**W-7895 Klettgau-Erzingen(DE)**

(74) Vertreter: **Blum, Rudolf Emil Ernst et al**
**c/o E. Blum & Co Patentanwälte Vorderberg**
**11**
**CH-8044 Zürich(CH)**

## Beschreibung

Es ist bekannt, dass Oberflächen aus Polymermaterialien oder anorganischen Materialien, wie Glas, dann, wenn sie mit einem Medium, insbesondere einem wässrigen flüssigen Medium, in Berührung kommen, das Proteine enthält, eine Schicht an Proteinmaterialen anlagern. Die Schichten an Proteinmaterialien sind nicht stabil, sondern sie bauen sich je nach der Proteinkonzentration in dem umgebenden flüssigen Medium auf oder ab und es findet ein Austausch der ursprünglich gebundenen Proteinmaterialien gegen andere Proteinmaterialien des umgebenden flüssigen Mediums statt. Die entsprechenden Proteinschichten sind ferner auch reaktiv, d.h. auf ihnen können sich weitere Komponenten ablagern. Besonders nachteilig ist dieser Effekt dort, wo entsprechende Polymermaterialien mit Körpergewebe und Blut in Berührung kommen, weil sich beispielsweise Blutplättchen und Fibrinogen an derartigen Proteinschichten anlagern und so Thromben entstehen können.

Auch in denjenigen Fällen, wo ein Kunststoff-Formkörper als Sensor oder Indikatorvorrichtung verwendet wird, die an seiner Oberfläche oder nahe seiner Oberfläche eine Substanz enthält, die zur Bestimmung von ionischen oder nicht ionischen Komponenten oder gelösten Gasen in einem flüssigen Medium geeignet ist, traten oft falsche Messergebnisse auf, wenn die entsprechende Bestimmung in einem Protein enthaltenden Medium durchgeführt werden sollte, wie zum Beispiel einer Körperflüssigkeit, insbesondere Blut, Blutplasma oder Blutserum. Bei dem Kontakt des Kunststoffes mit dem proteinhaltigen flüssigen Medium bildete sich auf der Oberfläche des Kunststoffes eine unstabile Proteinschicht aus, die ihre Belegungsdichte änderte, sobald sie mit Lösungen höheren Proteingehaltes oder niedrigen Proteingehaltes in Berührung kam. Diese sich ständig ändernde Belegungsdichte der Oberfläche mit Proteinen führte dazu, dass die zu bestimmende Komponente mehr Zutritt oder weniger Zutritt zu der an der Oberfläche des Kunstoffes befindlichen Indikatorkomponente hatte und somit bei konstanter Konzentration der zu bestimmenden Komponente unterschiedliche Messergebnisse erhalten wurden.

Bisher war man bestrebt, die oben genannten Schwierigkeiten, die sich bei der Ausbildung einer Proteinschicht auf Oberflächen von Polymermaterialien ergaben, zu beheben, indem man versuchte, die Proteinanlagerung an den Polymeroberflächen möglichst gering zu halten. Bisher wurde dies dadurch erreicht, indem man entweder solche Polymermaterialien verwendete, die völlig frei von irgendwelchen hydrophilen Gruppen waren oder andererseits solche Polymermaterialien auswählte, die sehr viele hydrophile Gruppen aufweisen, und die dann bei Kontakt mit wässrigen Medien sogenannte Hydrogele ausbildeten.

Ziel der vorliegenden Erfindung war es, die oben aufgezeigten Schwierigkeiten, die durch die Ausbildung einer Proteinschicht auf Polymermaterialien entstehen, nicht dadurch zu lösen, dass die entsprechende Proteinschicht möglichst dünn gehalten wird, sondern dadurch, dass die Ausbildung einer stabilen Proteinschicht erreicht wird. In dieser stabilen Proteinschicht soll die Konformation bzw. Konfiguration, also die räumliche Anordnung der Proteinmoleküle unverändert bleiben. Durch diese stabilisierte Konformation des Proteinmoleküls ist ein unveränderter Zutritt der in einem flüssigen Medium enthaltenen Komponenten zu der Oberfläche des Polymermaterials gewährleistet, während im Gegensatz dazu bei einer instabilen Proteinschicht, bei der sich die Konformation des angelagerten Proteinmoleküles ständig ändert, diese Zutrittsmöglichkeit einer Komponente aus dem flüssigen Medium zu der Oberfläche des Polymers ebenfalls verändert wird.

Ferner soll sich diese stabile Proteinschicht bei Kontakt mit wässrigen Medien in ihrer Belegungsdichte nicht ändern, und ausserdem soweit inaktiviert sein, dass eine Ablagerung von weiteren Komponenten, wie zum Beispiel Blutplättchen oder Fibrin, auf dieser stabilen Proteinschicht vermindert oder verhindert wird.

## BESCHREIBUNG DES STANDES DER TECHNIK

Aus der sehr grossen Anzahl an Veröffentlichungen, welche die Probleme der Verminderung der Anlagerung von Proteinschichten auf Kunststoffmaterialien erklären, sei auf die Monographie "Blood Surface Interactions" von J.P. Cazenaye et al., Verlag Elsevier, Amsterdam, New York, Oxford, 1986, verwiesen. In den Arbeiten von Chuang et al. (siehe beispielsweise J. Biomed. Mater. Res., 14, Seiten 467 ff, 1980) wird die Absorption von verschiedenen Proteinen auf Material mit der Markenbezeichnung "Cuprophane" mit derjenigen auf Polyvinylchlorid verglichen, beispielsweise die Absorption von $\alpha$-Thrombin, und es zeigte sich dabei, dass Polyvinylchlorid höhere Mengen absorbierte und die absorbierten Mengen ausserdem stark von der Proteinkonzentration im umgebenden Medium abhängig waren.

In der PCT Veröffentlichung WO84/01892 wird die Herstellung eines Materials beschrieben, das zur Verwendung als Blutgefässprothesen geeignet ist und auf dessen Oberfläche die Bindung und das Wachstum von Endothelzellen erleichtert ist. Vor der Verwendung dieses Prothesematerials wird dieses mit einem Peptid in Berührung gebracht, das sich von Elastin ableitet. Vorzugsweise besteht das Grundmaterial dieser Blutgefässprothesen aus einer menschlichen oder tierischen Vene oder Arterie oder einem Kunststoff aus der Gruppe der Polytetrafluoräthylene oder Polyäthylenterephthalate (nämlich Produkten mit der Markenbezeichnung Dacron), wobei diese Kunststoffe Hydroxylgruppen als Substituenten aufweisen.

In dieser Veröffentlichung wird ferner erläutert, dass die Innenwand der entsprechenden Blutgefässprothesen eine Beschichtung aus Elastin aufweisen soll, weil auf der Elastinschicht die Verankerung und das Wachstum von Endothelzellen erleichtert wird und damit die Bildung von Thromben verhindert wird.

Im dritten Absatz der Seite 6 dieser Veröffentlichung wird ferner erwähnt, dass Hydroxylgruppen in Tetrafluoräthylenpolymerisaten mit Hilfe von käuflich erhältlichen Aetzlösungen eingeführt werden können und dass auch an der Oberfläche von anderen Kunststoffmaterialien Sauerstoff enthaltende Gruppen, wie Hydroxylgruppen, Aldehydgruppen oder Carboxylgruppen chemisch eingeführt werden können. Der fraglichen Veröffentlichung ist jedoch kein Hinweis zu entnehmen, wie hoch die Anzahl der Sauerstoff enthaltenden Oberflächengruppierungen im Vergleich zu den unpolaren Oberflächengruppierungen des Polymermaterials sein soll, und das einzige Beispiel dieser Veröffentlichung betrifft ferner ein entsprechendes Prothesematerial, das nicht auf einer Kunststoffbasis beruht, sondern bei dem das Grundmaterial eine chemisch modifizierte Rinderarterie ist.

In der europäischen Patentveröffentlichung 0 201 378 werden Polymermaterialien oder Copolymere zur medizinischen oder chirurgischen Verwendung beschrieben, bei denen an die Polymerkette Carboxylgruppen direkt oder über einen aliphatischen Rest mit 1 - 15 Kohlenstoffatomen oder Phosphonsäuregruppen direkt oder über einen aliphatischen Rest mit 1 - 15 Kohlenstoffatomen oder Sulphonsäuregruppen direkt gebunden sein können. Gegebenenfalls liegen die entsprechenden Säuregruppen in Form ihrer Salze vor und diese hydrophil substituierten Polymermaterialien weisen entzündungshemmende Eigenschaften auf. Für den Fall, dass die hydrophilen Gruppen über eine -$CH_2$-Gruppe gebundene Carbonsäuregruppierungen oder deren Salze sind, weisen vorzugsweise mindestens 35 % der Monomereinheiten des Polymermaterials diese hydrophilen Substituenten auf. Aufgrund dieses hohen Gehaltes an hydrophilen Gruppen quellen die dort beschriebenen Produkte sehr stark, sobald sie mit einem wässrigen Medium in Berührung kommen, und es tritt dadurch beispielsweise eine Volumenvergrösserung von nahezu 100 % auf (siehe die Produkte des Beispiels 3).

Im Beispiel 5 wird ein modifiziertes Polystyrol beschrieben, bei dem mehr als 90 Mol-% der Monomerbestandteile im Benzolring eine Carboxylgruppe in Form ihres Natriumsalzes als Substituenten tragen.

In der fraglichen europäischen Patentveröffentlichung wird nicht erwähnt, dass die entsprechenden entzündungshemmenden Polymermaterialien zur Ausbildung einer stabilen Proteinschicht geeignet sind.

In der europäischen Patentveröffentlichung 0 061 312 werden biologisch verträgliche Polymermaterialien beschrieben, welche keine Thromben hervorrufen und an die Hauptkette dieser Polymermaterialien sind aliphatische Ketten mit 14 - 30 Kohlenstoffatomen covalent gebunden. Diese abstehenden Ketten stellen reversible Bindungsstellen für Albumin dar und es wird hervorgehoben, dass die Bindung des Albumins an dieses Polymermaterial ein dynamischer Prozess ist und dass beim Kontakt der entsprechenden Polymermaterialien mit Produkten, die reich an Albumin sind, wie Blut oder Blutserum, ein dynamischer Ersatz oder Austausch des gebundenen Albumins gegen frisches Albumin aus dem umgebenden flüssigen Miedium stattfindet.

In der europäischen Patentveröffentlichung 0 068 509 werden Copolymerisate beschrieben, die keine Bildung von Thromben hervorrufen, und in denen mindestens 5 Gew.-% der polymerisierbaren Monomerbestandteile eine Polyäthylenoxidkette aus mindestens 5 Aethylenoxideinheiten und eine polymerisierbare Kohlenstoff-Kohlenstoff-Doppelbindung aufweisen.

Bei einem Kontakt mit Wasser nehmen diese Copolymerisate 5 - 90 %, vorzugsweise 20 - 70 % Wasser auf. Es wird hervorgehoben, dass die dort beschriebenen hydrophilen Copolymerisate (im Gegensatz zu bereits früher bekannten Hydrogelen) zu keiner Anlagerung von Proteinen und Lipiden führen (siehe Seite 1, Zeile 21, bis Seite 2, Zeile 15, dieser Veröffentlichung).

In der europäischen Patentveröffentlichung 0 093 489 werden Copolymerisate aus Schwefelsäurestern des Vinylalkohols mit Acrylsäure, beziehungsweise Methacrylsäure, oder den Estern dieser ungesättigten Säuren beschrieben, wobei gegebenenfalls in dem Copolymerisat noch Einheiten an unverestertem Vinylalkohol enthalten sein können. Vorzugsweise bestehen 5 - 40 Mol-% der Monomereinheiten aus dem mit Schwefelsäure veresterten Vinylalkohol und 2 - 25 Mol-% der Monomereinheiten aus Acrylsäure, Methacrylsäure oder deren Estern. Diese stark hydrophil substituierten Copolymerisate mit Schwefelsäureestergruppierungen verhindern die Blutgerinnung infolge einer Inaktivierung von Thrombin. Es ist dieser Veröffentlichung kein Hinweis zu entnehmen, dass diese Copolymerisate in der Lage sind, an ihrer Oberfläche eine stabilisierte Proteinschicht auszubilden.

In der britischer Patentveröffentlichung 2 166 977A werden Formkörper zur medizinischen Anwendung beschrieben, die aus einem Vinylchloridkunststoff bestehen, der mit einer Zwischenschicht beschichtet ist, durch welche eine äussere Schicht aus vernetzter Gelatine auf diesem Kunststoff verankert wird. Bei einem Kontakt dieser Formkörper mit einem Protein enthaltenden Material kommt dementsprechend dieses nicht mit dem Polymermaterial aus Polyvinylchlorid, sondern nur mit der darauf verankerten Schicht aus vernetzter Gelatine in Berührung.

## BESCHREIBUNG DER ERFINDUNG

Ziel der vorliegenden Erfindung war es, Polymermaterialien zur Verfügung zu stellen, die in der Lage sind, nach dem Kontakt mit einem Protein enthaltenden flüssigen Medium an ihrer Oberfläche eine stabile Proteinschicht auszubilden. In dieser stabilen Proteinschicht soll die räumliche Anordnung der Proteinmoleküle unverändert bleiben und die Belegungsdichte der Proteinschicht soll sich bei einer nachfolgenden Behandlung der Polymeroberfläche in wässrigen Medien, die höhere oder tiefere Konzentrationen an Proteinen enthalten oder andere Proteine enthalten, als die zur Herstellung der stabilen Proteinschicht verwendeten Lösung, nicht ändern und es soll auch kein Austausch von Proteinmolekülen, die an der Oberfläche des Polymermaterials gebunden sind, gegen Proteinmoleküle in flüssigen Medien erfolgen, mit denen die Polymermaterialien während ihrer Verwendung in Berührung kommen.

Ueberraschenderweise zeigte es sich, dass die angestrebten Ziele durch ein Polymermaterial erreicht werden können, das in seinem Molekül ein bestimmtes Verhältnis an hydrophilen Gruppen, die Hydroxylgruppen oder Carboxylgruppen sind, zu lipophilen unpolaren oder wenig polaren Gruppierungen aufweist.

Ein Gegenstand der vorliegenden Erfindung ist daher ein Polymermaterial, das hydrophile Substituenten trägt und das nach einem Kontakt mit einem Proteine enthaltenden flüssigen Medium eine stabile Proteinschicht anlagert und dadurch gekennzeichnet ist, dass das Polymermaterial ein Copolymeres ist, das Monomereinheiten aufweist, die von Alkenen oder Alkinen mit einer oder mehr Mehrfachbindungen stammen, wobei diese Alkene und Alkine gegebenenfalls einen oder mehrere Substituenten tragen, die Chloratome, Bromatome, Arylreste, Carbonsäureestergruppen, Carbonsäureamidgruppen, Nitrilgruppen, veresterte oder verätherte Hydroxylgruppen, Ketogruppen, freie oder acetalisierte Aldehydgruppen sind, und/oder das Copolymerisat Polyesteranteile, Polyurethananteile, Polyamidanteile und/oder Polycarbonatanteile enthält, und wobei 5 - 25 Mol-% der Monomereinheiten des Copolymerisates ein oder mehrere hydrophile Substituenten tragen, die Hydroxylgruppen und/oder Carboxylgruppen sind.

Vorzugsweise sind in den erfindungsgemässen Polymermaterialien die hydrophilen Hydroxylgruppen, bzw. Carboxylgruppen entweder direkt an ein Kohlenstoffatom der Hauptkette des Polymermaterials gebunden oder an die Hauptkette des Polymermaterials über eine aliphatische, cycloaliphatische oder aromatische Seitenkette mit maximal 6 Kohlenstoffatomen gebunden. Die erfindungsgemässen Polymermaterialien sollen also vorzugsweise im wesentlichen lineare Copolymerisate sein, bei denen von der Polymerkette keine längeren Seitenketten abstehen. Speziell bevorzugt sollen die hydrophilen Hydroxylgruppen, bzw. Carboxylgruppen direkt an Kohlenstoffatome der Polymerhauptkette gebunden sein. Dieses strukturelle Merkmal der entsprechenden Polymermaterialien dürfte wesentlich zu der Ausbildung der stabilen Proteinschicht an der Oberfläche des Polymermaterials beitragen.

Es konnte ferner gezeigt werden, dass es wesentlich ist, dass 5 - 25 Mol-% der Monomereinheiten des Copolymerisates eine oder mehrere Hydroxylgruppen und/oder Carboxylgruppen, vorzugsweise eine Hydroxylgruppe oder Carboxylgruppe, als hydrophilen Substituenten tragen.

Mit entsprechenden Polymermaterialien, die geringere Anteile oder höhere Anteile an Hydroxylgruppen, bzw. Carboxylgruppen aufweisenden Monomerbestandteilen besassen, konnten die angestrebten Ziele nicht erreicht werden.

Zu diesem Zwecke wurden die erfindungsgemässen Polymermaterialien mit solchen verglichen, die analog aufgebaut waren, wobei jedoch weniger als 5 % der Monomereinheiten des Copolymerisates die genannten hydrophilen Substituenten trugen, beziehungsweise mit entsprechenden Polymermaterialien, die frei von hydrophilen Substituenten waren. Ueberraschenderweise zeigte es sich, dass bei den erwähnten Polymermaterialien zu Vergleichszwecken im allgemeinen weniger Protein angelagert wurde als bei den erfindungsgemässen Polymermaterialien, dass jedoch die gebildete Proteinschicht nicht stabil war. Erfindungsgemässe Polymerisate und entsprechende weniger hydrophile Gruppen tragende oder von hydrophilen Gruppen freie Polymermaterialien zu Vergleichszwecken wurden jeweils mit einer Puffer enthaltenden 7 Gew.-%igen Rinderserumalbuminlösung behandelt, biskeine weitere Ablagerung von Protein an den Polymeroberflächen mehr feststellbar war. Anschliessend wurden die mit der Proteinschicht beladenen Polymermaterialien mit einer physiologischen Kochsalzlösung in Berührung gebracht. Diese Behandlung änderte bei den erfindungsgemäss zu verwendenden Polymermaterialien die stabilisierte Proteinschicht nicht, während bei den Polymermaterialien zu Vergleichszwecken das Protein der Proteinschicht von der Kochsalzlösung ausgewaschen wurde.

Analoge Vergleichsversuche wurden auch mit solchen Polymermaterialien durchgeführt, die einen höheren Anteil an hydrophilen Gruppen aufwiesen als die erfindungsgemässen Polymermaterialien. Diese Vergleichsversuche wurden also mit Copolymerisaten durchgeführt, in denen mehr als 25 Mol-% der Monomereinheiten des Copolymerisates einen oder mehrere der genannten hydrophilen Substituenten trugen. Auch diese Copolymerisate zu Vergleichszwecken wurden nach ihrer Behandlung mit einer 7 Gew.-%igen wässrigen, Puffer enthaltenden Rinderserumalbuminlösung einer Nachbehandlung mit physiologischer Kochsalzlösung unterzogen. Ueberraschenderweise zeigte es sich, dass auch in diesem Falle durch die Nachbehandlung die gebildete Proteinschicht von dem Copolymerisat wieder entfernt wurde.

Copolymerisate zu Vergleichszwecken, in welchen mehr als 25 Mol-% der Monomereinheiten eine oder mehrere Hydroxylgruppen oder Carboxylgruppen als Substituenten tragen, besitzen ferner den Nachteil, dass sie bei einem Kontakt mit wässrigen Medien stark quellen. Polymermaterialien mit deutlich höheren Anteilen an hydrophilen Substituenten als die erfindungsgemässen Polymermaterialien weisen im allgemeinen bei Kontakt mit wässrigen Lösungen eine Volumensvergrösserung von 20 - 50 %, bezogen auf das Volumen des Polymermaterials vor dem Kontakt mit der wässrigen Lösung, auf.

Im Gegensatz dazu quellen die erfindungsgemässen Polymermaterialien bei einem Kontakt mit Wasser oder wässrigen Lösungen kaum oder sie quellen nur in einem geringen Ausmass. Vorzugsweise tritt bei dem Kontakt der erfindungsgemässen Polymermaterialien mit Wasser oder wässrigen Lösungen eine Volumensvergrösserung um höchstens 15 %, bezogen auf das Volumen des Polymermaterials vor dem Kontakt mit Wasser oder der wässrigen Lösung, auf. Bezogen auf das Gewicht des Polymermaterials vor und nach dem Kontakt mit wässrigen Lösungen beträgt die Gewichtsvergrösserung durch Quellung im allgemeinen höchstens 18 Gew.%

Vorzugsweise weisen die erfindungsgemässen Polymermaterialien jedoch eine noch geringere Quellung auf. Im allgemeinen tritt bei Kontakt mit Wasser oder wässrigen Lösungen höchstens eine Volumensvergrösserung um 10 Vol.%, vorzugsweise höchstens eine Volumensvergrösserung von 8 Vol.%, bezogen auf das Volumen des entsprechenden Polymermaterials vor dem Kontakt mit Wasser oder der wässrigen Lösung, auf.

Die vorliegende Erfindung soll nicht durch irgendwelche theoretischen Ueberlegungen eingeschränkt werden. Dennoch könnte eventuell vermutet werden, dass bei den erfindungsgemässen Copolymerisaten an der Oberfläche derselben ein ausgewogenes Verhältnis von hydrophilen und hydrophoben Gruppen zur Verfügung steht, sodass sowohl hydrophile Gruppen des Proteinmoleküls als auch hydrophobe Gruppen des Proteinmoleküls in dieser Oberflächenschicht verankert werden und so eine starke Fixierung der Proteinmoleküle an der Oberfläche stattfindet. Dadurch dass sowohl hydrophile Gruppen des Proteinmoleküls als auch hydrophobe Gruppen des Proteinmoleküls an die entsprechenden Gruppen der Oberfläche des Copolymerisates gebunden sind, dürfte auch die stabile räumliche Anordnung der Proteinmoleküle an der Oberfläche erreicht werden, also die unveränderte Konformation der in der Proteinschicht gebundenen Proteinmoleküle.

Wie bereits erwähnt wurde, wiesen Polymermaterialien, die bisher für einen Kontakt mit biologischem Protein enthaltenden Material vorgesehen wurden, im allgemeinen entweder sehr stark hydrophile Eigenschaften oder ausschliesslich hydrophobe Eigenschaften auf, sodass an der Oberfläche derartiger Polymermaterialien entweder im wesentlichen hydrophile Gruppen vorlagen, an denen nur die hydrophilen Gruppen von Proteinmolekülen gebunden wurden oder diese Polymermaterialien wiesen an der Oberfläche im wesentlichen nur hydrophobe Gruppen auf, an denen nur die hydrophoben Gruppierungen der Proteinmoleküle gebunden wurden. In beiden Fällen blieben dann ungebundene Proteinkettenenden frei vorhanden, die die Anlagerung von weiteren Bestandteilen, wie zum Beispiel Blutplättchen und Fibrin, begünstigen. Treten jedoch sowohl hydrophile Gruppen eines Proteinmoleküles als auch hydrophobe Gruppen desselben mit den entsprechenden Gruppen an der Oberfläche des Polymermateriales in Wechselwirkung, dann kann angenommen werden, dass dadurch nicht nur eine Stabilisierung des Proteinmoleküls an der Oberfläche erfolgt, sondern auch reaktive freistehende Proteinkettenenden, welche die Anlagerung von weiteren Komponenten, wie Blutplättchen und Fibrin, begünstigen, nicht mehr zur Verfügung stehen.

Die stabile Konformation der an die erfindungsgemässen Polymermaterialien gebundenen Proteinschicht konnte spektroskopisch nachgewiesen werden. Die dünnen an die Oberfläche der Polymermaterialien gebundenen Proteinschichten wurden hiezu mit Hilfe der polarisierten ATR Spektroskopie (Attenuated Total Reflection Spectroscopy) untersucht und es zeigte sich dabei, dass die Position der Carbonylgruppen der Säureamidgruppierung der gebundenen Proteinmoleküle unverändert bleibt und somit auch die räumliche Anordnung dieser Proteinmoleküle in der Proteinschicht stabilisiert ist.

Ein weiterer Gegenstand der vorliegenden Erfindung ist die Verwendung der Polymermaterialien, die befähigt sind, eine stabile Proteinschicht anzulagern zur Herstellung von Formkörpern, bzw. Beschichtungen, die bei ihrem Gebrauch mit einem Protein enthaltenden flüssigen Medium oder einem Säugergewebe in Berührung kommen.

Wenn entsprechende Formkörper mit einem Protein enthaltenden flüssigen Medium in Berührung kommen, wie zum Beispiel biologischen Flüssigkeiten, wie Blut, Blutserum oder Blutplasma, oder wenn sie mit einem lebenden Säugergewebe in Berührung kommen, dann bildet sich an den Oberflächen, die mit den Proteinen in Kontakt stehen, eine stabilisierte Proteinschicht aus, die sich bei Kontakt mit wässrigen Medien in ihrer räumlichen Anordnung nicht ändert, und ferner wird diese stabilisierte Proteinschicht bei einem Kontakt mit wässrigen Medien nicht von der Polymeroberfläche entfernt und auch nicht gegen Proteine ausgetauscht, die in den wässrigen Medien enthalten sind, welche mit dieser Oberflächenschicht in Berührung kommen.

Die erfindungsgemässen Polymermaterialien, beziehungsweise die aus ihnen hergestellten Formkörper, sind vorzugsweise Polyvinylhalogenidcopolymerisate oder Polyvinylidenhalogenidcopolymerisate, in welchen die Halogenatome Chloratome und/oder Bromatome sind, und in welchen 5 - 25 Mol-% der Monomereinheiten eine oder mehrere Hydroxylgruppen und/oder Carboxylgruppen als hydrophile Substituenten tragen. Vorzugsweise tragen diese hydrophil substituierten Monomereinheiten eine hydrophile Gruppe, die eine Hydroxylgruppe oder Carboxylgruppe, vorzugsweise eine Hydroxylgruppe, ist. Falls in diesen Polyvinylhalogenidcopolymerisaten oder Polyvinylidenhalogenidcopolymerisaten noch weitere Substituenten tragende Monomereinheiten enthalten sind, dann sind die entsprechenden Substituenten vorzugsweise Estergruppierungen, Säureamidgruppierungen, Nitrilgruppierungen, Carbonatgruppierungen und/oder Urethangruppierunger. Gegebenenfalls können in diesen Polyvinylhalogenid- bzw. Polyvinylidenhalogenid-copolymerisaten auch noch aromatische Gruppierungen vorhanden sein, beispielsweise solche, die von Styrolmonomeren oder substituierten Styrolmonomeren stammen.

In diesen Polyvinylchlorid-, bzw. bromidcopolymerisaten und Polyvinylidenchlorid-, bzw. bromidcopolymerisaten sind vorzugsweise 7 - 19 Mol-%, speziell bevorzugt 8 - 17 Mol-%, und insbesondere 10 - 14 Mol-%, solche Monomereinheiten, die eine Hydroxylgruppe oder allenfalls eine Carboxylgruppe als Substituenten tragen.

Speziell bevorzugte erfindungsgemässe Copolymerisate, beziehungsweise in den erfindungsgemässen Formkörpern enthaltene Copolymerisate, sind solche aus Vinylchlorid und Vinylalkohol, und in diesen Copolymerisaten stammen vorzugsweise 7 - 19, im allgemeinen 8 - 17 Mol-% der Monomereinheiten aus Vinylalkohol. Gegebenenfalls können in diesen Vinylchlorid-Vinylalkohol-Copolymerisaten noch Monomereinheiten vorhanden sein, die Estergruppen als Substituenten tragen, vorzugsweise veresterte Hydroxylgruppen.

Die entsprechenden Polymermaterialien aus Vinylchlorid und Vinylalkohol können hergestellt werden, indem man ein entsprechendes Copolymerisat aus Vinylchlorid und einem veresterten Vinylalkohol, beispielsweise Vinylacetat, verseift, bis eine entsprechende Anzahl der veresterten Hydroxylgruppen in freie Hydroxylgruppen übergeführt ist, sodass in dem entsprechenden Endprodukt 7 - 19, beispielsweise 8 - 17 Mol-% der Monomereinheiten von Vinylalkohol stammen.

In speziell bevorzugten Copolymerisaten aus Vinylchlorid und Vinylalkohol stammen 11 - 14 Mol-% der Monomereinheiten von Vinylalkohol, und insbesondere bestehen 12,8 - 13,3 Mol-% der Monomereinheiten aus Vinylalkohol.

Die erfindungsgemässen Polymermaterialien, bzw. die aus ihnen hergestellten Formkörper, können gegebenenfalls als weitere Komponente einen Weichmacher enthalten. Bevorzugt sind dabei solche Weichmacher enthaltende Copolymerisate, bzw. entsprechende Formkörper, in denen der Anteil an Weichmacher ziemlich hoch ist, und im allgemeinen enthält die Zusammensetzung aus Copolymerisat und Weichmacher einen gewichtsmässig gleichen oder höheren Anteil an Weichmacher als Copolymerisat. Speziell bevorzugt enthalten entsprechende weichgemachte Polymermaterialien, Beschichtungen und Formkörper 30-50 Gew.-% an dem Copolymerisat und 50 - 70 Gew.-% an dem Weichmacher, jeweils bezogen auf das Gesamtgewicht der Zusammensetzung aus Copolymerisat und Weichmacher. Speziell bevorzugt enthalten die Zusammensetzungen aus Copolymerisat und Weichmacher 30 - 36 Gew.-% an dem Copolymerisat und 64 - 70 Gew.-% an einem Weichmacher, jeweils bezogen auf das Gesamtgewicht.

Bevorzugte Weichmacher sind Esterweichmacher, insbesondere Diester von Dicarbonsäure und Tetraester von Tetracarbonsäure, in denen die Alkoholkomponente ein Alkanol mit 5 - 15 Kohlenstoffatomen ist. Typische Beispiele sind die Diester der Adipinsäure oder der Sebacinsäure mit Alkanolen von 7 - 9 Kohlenstoffatomen.

Auch in entsprechenden, Weichmacher enthaltenden Polymermaterialien, Formkörpern oder Oberflächenbeschichtungen von anderen Substraten, ist das bevorzugte hydrophile Gruppen enthaltende Copolymerisat ein entsprechendes Copolymerisat aus Vinylchlorid und Vinylalkohol, insbesondere ein Copolymerisat der weiter vorne angegebenen Zusammensetzungen bezüglich der Gehalte an Monomereinheiten, die von Vinylalkohol stammen.

Vorzugsweise liegen die erfindungsgemässen Polymermaterialien als Formkörper oder Beschichtungen von verschiedenen Substraten vor, wobei bei der Verwendung der Formkörper oder der mit der Beschichtung versehenen Substrate mindestens ein Teil der Oberfläche derselben mit einem proteinhaltigen flüssigen Medium oder einem Gewebe von Säugetieren in Berührung kommt.

Entsprechende Formkörper oder beschichtete Substrate werden in sehr verschiedenen Einsatzbereichen verwendet, in denen es von ausschlaggebender Bedeutung ist, dass auf der Oberfläche dieser Produkte, die mit dem proteinhaltigen Medium oder einem lebenden Gewebe von Säugetieren in Berührung kommt, eine stabile Proteinschicht gebildet wird, und bei denen ferner verhindert werden soll, dass auf einer an der Oberfläche gebildeten Proteinschicht die Abscheidung weiterer Substanzen, wie zum Beispiel Blutplättchen oder Blutplasma, begünstigt wird.

In all diesen Einsatzgebieten sind erfindungsgemässe Formkörper oder Oberflächenbeschichtungen auf anderen inerten Substraten, beispielsweise auf anorganischen Materialien, wie Glas, Keramik, Metall oder organischen Materialien, wie die verschiedensten Polymermaterialien, gegenüber bisher zu diesem Zweck eingesetzten Produkten vorteilhaft.

Als Beispiele für entsprechende Produkte seien genannt: Behälter oder leitungsartige Gebilde, die mit proteinhaltigen Medien in Berührung kommen, wie zum Beispiel mit biologischen Flüssigkeiten, wie Gesamtblut, Blutplasma oder Blutserum. Entsprechende Behälter können beispielsweise zur Aufbewahrung der biologischen Flüssigkeiten, zur Durchleitung der biologischen Flüssigkeiten, zum Beispiel bei der Blutentnahme oder Blutinfusion, und zur Untersuchung der biologischen Flüssigkeiten verwendet werden, beispielsweise Glasröhrchen oder Kunststoffröhrchen, in denen auf die Anwesenheit von Komponenten im Blutserum getestet wird, wie zum Beispiel Antikörper, Hormone, Insulin, Gallenbestandteile, Harnstoff, toxische Stoffe, Medikamente, Dopingmittel, ionische Bestandteile und Aehnliches.

Bei entsprechenden bisher verwendeten Behältern oder leitungsartigen Gebilden traten Schwierigkeiten auf, weil sich an der Oberfläche, die mit der biologischen Flüssigkeit in Berührung kam, Proteinschichten ausbildeten, die instabil waren und in denen sich die Konformation der Proteinmoleküle der Proteinschicht ständig änderte. Ferner änderte sich bei entsprechenden bisher verwendeten Behältern oder leitungsartigen Gebildendie Belegungsdichte der Proteinschicht in Abhängigkeit von der biologischen Flüssigkeit, mit der diese Behälter oder Leitungen in Berührung standen. Diese Unstabilität der Proteinschicht bezüglich ihrer Konformation und ihrer Belegungsdichte führte insbesondere auch bei Teströhrchen aus Quarz, Glas oder Kunststoff, dann zu einer Verfälschung der Messergebnisse, wenn optische Verfahren angewandt wurden, beispielsweise eine Bestimmung der Verschiebung der Absorption im sichtbaren, Infrarot- oder Ultraviolettbereich oder eine Entstehung oder Lösung einer Fluoreszenz oder Bestimmung von anderen optischen Eigenschaften, wie beispielsweise des Brechungsindexes.

Ein weiteres Einsatzgebiet von erfindungsgemässen Formkörpern oder mit einer erfindungsgemässen Beschichtung versehenen Formkörpern liegt im Bereiche, wo diese mit lebendem, tierischem Gewebe in Berührung gebracht werden. Als Beispiele seien die verschiedensten Typen an Kathetern, beschichtete metallische oder keramische Werkstoffe, wie sie beispielsweise in künstlichen Gelenken eingesetzt werden, röhrenförmige Leitungssysteme für Körperflüssigkeiten, wie Blut, Lymphe, Harn und Aehnliches, Kunststoffe für die Herzchirurgie, elastische Materialien z.B. für künstliche Sehnen, sowie Kontaktlinsen, genannt. In diesen Einsatzgebieten sind mit den erfindungsgemässen Polymermaterialien beschichtete entsprechende Substrate gegenüber den jeweiligen unbeschichteten Substraten von Vorteil. Dies gilt auch für aus den erfindungsgemässen Kunststoffen bestehende künstliche Leitersysteme, Sehnen, Organe oder Organteile. Im Fall von beschichteten Substraten ist es speziell vorteilhaft, dass die erfindungsgemässen, hydrophile Gruppen aufweisenden Polymerisate eine sehr gute Haftung auf den meisten Substratmaterialien aufweisen, insbesondere dann, wenn die entsprechenden Polymermaterialien als weitere Komponenten noch Weichmacher enthalten.

In all den oben genannten Fällen können die erfindungsgemässen Formkörper, beziehungsweise die erfindungsgemässen Beschichtungen von konventionellen Substraten vor ihrer Verwendung mit einer an der Oberfläche der Formkörper, beziehungsweise Beschichtungen relativ fest verankerten Proteinschicht versehen werden. Diese auf der Oberfläche des Copolymerisates befindlichenstabilen Proteinschichten ändern ihre Konformation und/oder ihre Belegungsdichte nicht, wenn die entsprechenden Polymerformkörper, bzw. Polymerschichten mit einem wässrigen Medium in Kontakt kommen, das gegebenenfalls gelöste Salze und/oder Proteine enthält. Im zuletzt genannten Fall erfolgt auch kein Austausch der auf der Polymeroberfläche vor der Verwendung verankerten Proteine gegen die jeweiligen Proteine des umgebenden wässrigen Mediums.

Ein weiterer Gegenstand der vorliegenden Erfindung ist die Verwendung des in Form von Formkörpern oder Beschichtungen vorliegenden erfindungsgemässen Polymermaterials, die dadurch gekennzeichnet ist, dass man bei der Verwendung mindestens eine Oberfläche des Formkörpers oder der Beschichtung mit einem proteinhaltigen flüssigen Medium oder einem Gewebe von Säugetieren in Berührung bringt, wobei sich dadurch auf dieser Oberfläche des Formkörpers oder der Beschichtung eine Proteinschicht ausbildet, die an dem Polymermaterial so stark verankert ist, dass sie bei einem Kontakt mit einem wässrigen Medium, das gegebenenfalls Proteine und/oder gelöste Salze enthält, nicht von der Polymeroberfläche entfernt und/oder gegen Fremdproteine ausgetauscht wird.

Gemäss einer bevorzugten Verwendungsart sind die verwendeten Formkörper und/oder Beschichtungen Sensoren oder Indikatorvorrichtungen, die zur Bestimmung von ionischen oder nichtionischen Komponenten oder gelösten Gasen in einem wässrigen proteinhaltigen Medium dienen, wobei in diesem Fall an der Oberfläche oder den Bereichen nahe der Oberfläche des Sensors oder der Indikatorvorrichtung, die bei der Bestimmung der ionischen, beziehungsweise nichtionischen Komponenten oder der gelösten Gase in dem wässrigen proteinhaltigen Medium mit diesem in Berührung kommt, ein Indikator enthalten ist, der eine Eigenschaft ändert, sobald er mit der zu bestimmenden Komponente, beziehungsweise dem gelösten Gas, in Berührung kommt oder der in einem zweiten Indikator eine derartige Aenderung hervorruft.

Derartige Formkörper oder Beschichtungen können als Indikator eine Substanz enthalten, die bei Anwesenheit der zu bestimmenden Komponente oder des gelösten Gases in dem wässrigen proteinhaltigen Medium ihre Absorption im sichtbaren, infraroten oder ultravioletten Lichtbereich ändert oder das Auftreten oder das Verschwinden einer Fluoreszenz zeigt oder der Sensor kann ein Biosensor sein, der eine biologisch wirksame Komponente, beispielsweise ein Enzym, ein Antigen, einen Antikörper oder einen Mikroorganismus enthält, wobei bei der Reaktion mit der im wässrigen proteinhaltigen Medium zu bestimmenden Komponente eine Wärmeentwicklung, eine Farbveränderung, eine Niederschlagsbildung, eine Aenderung des pH-Wertes, eine Auflösung eines Niederschlages oder eine solche Veränderung stattfindet, die mit Hilfe eines weiteren Indikators festgestellt werden kann. Die Aenderung, die auftritt, wenn der Indikator oder der Biosensor mit der im wässrigen proteinhaltigen Medium zu bestimmenden Komponente in Berührung kommt, kann auch eine Aenderung der elektrischen Leitfähigkeit oder der Wärmeleitfähigkeit sein.

Als Beispiele für entsprechende Indikatorvorrichtungen seien Teststreifen. Formkörper. beispielsweise kleine Kügelchen, oder ähnliche Materialien genannt, die aus einem erfindungsgemässen Polymerisat bestehen, oder an der Oberfläche eine Beschichtung aus dem erfindungsgemässen Polymermaterial tragen, wobei sich an der Oberfläche oder einer Oberflächenschicht als weitere Komponente ein Indikator oder eine Substanz befindet, die in Kombination mit einem zweiten Indikator eine Aenderung erfährt, sobald sie mit der zu bestimmenden Komponente in Berührung kommt.

Entsprechende Sensoren können auch in schwer zugänglichen Stellen des lebenden Organismus eingeführt werden, beispielsweise in den Magen-Darm-Trakt oder in Blutgefässe, wobei dann über einen Transduktor, wie zum Beispiel einen Lichtleiter, die entsprechenden Signale übermittelt werden und dann von aussen die Beobachtungen durchgeführt werden, beispielsweise mit Hilfe eines Bildschirmes. Die Anwendungsgebiete für solche Sensoren, beziehungsweise Optoden sind sehr vielseitig und die an der Oberfläche oder in der Oberflächenschicht des Sensors enthaltene Indikatorkomponente erlaubt die Feststellung verschiedenster Komponenten in dem sie umgebenden Medium.

In all den genannten Fällen von Sensoren, Optoden, Indikatorstreifen und ähnlichen Vorrichtungen traten bisher Fehler auf, weil die Oberfläche in der oder an der sich die Indikatorsubstanz befand, durch sich ständig ändernde Proteinschichten belegt wurde und dadurch bei gleicher Konzentration oder Aktivität der zu bestimmenden Komponente diese unterschiedlichen Zutritt zu der Indikatorkomponente im Sensor oder der Indikatorvorrichtung hatte und damit unterschiedliche Messergebnisse geliefert wurden.

Entsprechende erfindungsgemässe Vorrichtungen gewährleisten durch die stabilisierte Proteinschicht auf ihrer Oberfläche ein konstantes Messergebnis bei konstanter Konzentration oder Aktivität der zu bestimmenden Komponente, und zwar unabhängig von einer wechselnden und sich rasch ändernden Proteinkonzentration in dem umgebenden Medium.

Die erfindungsgemässen Polymermaterialien, beziehungsweise die Polymermaterialien der erfindungsgemässen Formkörper oder Beschichtungen können nach bekannten Verfahren der Polymerisation der entsprechenden Monomerbestandteile hergestellt werden.

Vorteilhafter ist es jedoch in allgemeinen die Copolymeren, die Monomereinheiten aufweisen, die von Alkenen oder Alkinen mit einer oder mehr Mehrfachbindungen stammen, wobei diese Alkene und Alkine gegebenenfalls einen oder mehrere Substituenten tragen, die Chloratome, Bromatome, Arylreste, Carbonsäureestergruppen, Carbonsäureamidgruppen, Nitrilgruppen, veresterte oder verätherte Hydroxylgruppen, Ketogruppen, freie oder acetalisierte Aldehydgruppen sind, und/oder die Copolymeren, die Polyesteranteile, Polyurethananteile, Polyamidanteile und/oder Polycarbonatanteile enthalten, wobei 5 - 25 Mol-% der Monomereinheiten des Copolymerisates ein oder mehrere hydrophile Substituenten tragen, die Hydroxylgruppen und/oder Carboxylgruppen sind, dadurch herzustellen, dass man aus einem entsprechenden Homopolymerisat oder Copolymerisat, in welchem die Hydroxylgruppen in veresterter Form und/oder die Carboxylgruppen, in veresterter Form, in Form von Säureamiden oder als Nitril vorliegen, die Hydroxylgruppen, bzw. Carboxylgruppen durch Verseifung freisetzt, bis in dem Copolymerisat der Anteil an Monomereinheiten, die ein oder mehr hydrophile Substituenten aufweisen, welche Hydroxylgruppen und/oder Carboxylgruppen sind, 5 - 25 Mol-% beträgt.

Wenn man nach dem oben beschriebenen Verfahren ein Polyvinylhalogenidcopolymerisat oder ein Polyvinylidenhalogenidcopolymerisat herstellt, in welchem die Halogenatome Chloratome oder Bromatome sind und in welchem 5 - 25 Mol-% der Monomereinheiten dieses Copolymerisates ein oder mehrere hydrophile Substituenten tragen, die Hydroxylgruppen und/oder Carboxylgruppen sind, dann setzt man aus einem entsprechenden Polyvinylhalogenidcopolymerisat, beziehungsweise Polyvinylidenhalogenidcopolymerisat, in welchem die Hydroxylgruppen in veresterter Form und die Carboxylgruppen in veresterter Form oder in Form von Säureamiden oder als Nitril vorliegen, die entsprechenden Hydroxylgruppen, beziehungsweise Carboxylgruppen, durch Verseifung frei, bis in dem Vinylhalogenid-copolymerisat, bzw. Vinylidenhalogenid-copolymerisat, der Anteil an Monomereinheiten, die eine oder mehr Hydroxylgruppen und/oder Carboxylgruppen als Substituenten aufweisen, 5 - 25 Mol-%, vorzugsweise 7 - 20 Mol-%, beträgt.

Die speziell bevorzugten Copolymerisate aus Vinylchlorid und Vinylalkohol, in denen 7 - 20 Mol-% der Monomereinheiten, vorzugsweise 11 - 14 Mol-% oder Monomereinheiten, des Copolymerisates aus Vinylalkohol stammen, können beispielsweise hergestellt werden, indem man in einem Copolymerisat aus Vinylchlorid und Vinylacetat, mit einem Minimalgehalt von 7 Mol-% an Vinylacetatmonomereinheiten durch Verseifung die Acetatgruppen abspaltet, bis in dem Copolymerisat der Gehalt an Vinylalkoholmonomereinheiten in dem angeführten Bereich liegt. Gegebenenfalls kann das nach der Verseifung erhaltene Copolymerisat in seiner Struktur noch Monomereinheiten aufweisen, die von Vinylacetat stammen.

Wenn das erfindungsgemässe Polymermaterial, beziehungsweise das Polymermaterial der erfindungsgemässen Formkörper oder Beschichtungen ein Copolymerisat aus Polyvinylchlorid und Polyvinylalkohol ist, in dem 5 Mol-% der Monomereinheiten Vinylalkoholeinheiten sind, dann weist dieses Produkt aufgrund seiner Zusammensetzung aus 95 Mol-% Polyvinylchlorid, dessen Molekulargewicht 62,5 beträgt, und 5 Mol-% Polyvinylalkohol, dessen Molgewicht 44 beträgt, ein durchschnittliches Molekulargewicht der Monomereinheiten von 61,57 auf. Der Gehalt an Hydroxylgruppen beträgt in diesem Produkt dementsprechend 1,38 Gew.-%.

Wenn das erfindungsgemässe Polymermaterial, beziehungsweise das Polymermaterial der erfindungsgemässen Formkörper oder Beschichtungen ein Copolymerisat aus Polyvinylchlorid und einer ungesättigten Carbonsäure der Formel $C_2H_3$-COOH ist und 5 Mol-% der Monomerbestandteile von dieser ungesättigten Monocarbonsäure stammen, dann weist dieses Produkt aufgrund des Molekulargewichtes der MonocarbonsäureMonomereinheit von 72,06 ein durchschnittliches Molekulargewicht der Monomereinheiten von 63,0 auf und dementsprechend einen Gehalt an COOH-Gruppen von 3,57 Gew.-% auf.

Ein carboxylsubstituiertes Polyvinylchlorid mit einem wesentlich geringeren Gehalt an Carboxylgruppen, nämlich einem Gehalt von nur 1,7 Gew.-% ist im Handel von der Firma Aldrich erhältlich und durch Reduktion der Carboxylgruppen dieses Produktes wurde ein hydroxysubstituiertes Polyvinylchlorid hergestellt, dessen Sauerstoffgehalt 0,7 Gew.-% beträgt. Die Herstellung dieses hydroxysubstituierten Polyvinylchlorides wird in der Veröffentlichung von Trevor Satchwill und D. Jed Harrison im J. Electroanal. Chem 202 (1986) auf Seiten 75 - 81 beschrieben. Dieses Polymermaterial wurde ebenfalls getestet und es zeigte sich, dass es nicht in der Lage war, an seiner Oberfläche eine stabile Proteinschicht auszubilden, nachdem es mit einer Rinderserumalbumin enthaltenden Lösung in Berührung gebracht worden war. Die gebildete Proteinschicht konnte anschliessend mit Hilfe von physiologischer Kochsalzlösung wieder abgewaschen werden. Auch die weiter vorne beschriebene spektroskopische Untersuchung der mit der Proteinschicht belegten Polymeroberfläche zeigte, dass sich in diesem Fall die Konformation der Proteinmoleküle ständig änderte, also die Lage der Carbonylgruppen der Säureamidgruppierungen des Proteines nicht unverändert blieb.

Zu Vergleichszwecken wurden ferner Polyvinylchloridcopolymerisate hergestellt, in welchen jedoch nur 3 Mol-% der Monomereinheiten von Polyvinylalkohol, beziehungsweise von einer ungesät-

tigten Monocarbonsäure der Formel $C_2H_3$-COOH stammten. Auch in diesem Fall war nach der Behandlung mit einer Rinderserumalbumin enthaltenden wässrigen Lösung die gebildete Proteinschicht an der Oberfläche des Polymermaterials nicht stabilisiert. Sie konnte mit physiologischer Kochsalzlösung wieder abgewaschen werden und die Konformation der Proteinmoleküle dieser Oberflächenschicht veränderte sich.

Aus diesen Vergleichsversuchen sieht man also, dass Polymermaterialien, deren Gehalt an hydrophilen Gruppen unterhalb des Bereiches der erfindungsgemäss zu verwendenden Polymermaterialien liegt, nicht zu den gewünschten Erfolgen führen.

Die erfindungsgemässen Polymermaterialien, beziehungsweise die Copolymerisate der erfindungsgemässen Formkörper oder Beschichtungen müssen ein hohes Molekulargewicht aufweisen, im allgemeinen ein Molekulargewicht von mindestens 10'000, vorzugsweise mindestens 40'000, und im allgemeinen über 50'000, beispielsweise bei 90'000 oder noch höher. Der K-Wert der Polymermaterialien soll über 50 liegen.

Die Erfindung sei nun anhand von Beispielen näher erläutert:

Beispiel 1

Herstellung eines Copolymeren aus Vinylalkohol und Vinylchlorid

Das Ausgangsmaterial für die Herstellung dieses Copolymerisates war ein Copolymerisat aus 82,9 Gew.-% Vinylchlorid und 17,1 Gew.-% Vinylacetat. Aufgrund des Molekulargewichtes von Vinylchlorid von 62,5 und des Molekulargewichtes von Vinylacetat von 86,09, beträgt die molare Zusammensetzung dieser Copolymerisate 86,975 Mol-% Vinylchlorid und 13,025 Mol-% Vinylacetat.

5 g dieses Copolymeren wurden in 100 ml Tetrahydrofuran gelöst und diese Lösung wurde tropfenweise während zehn Minuten zu 30 ml einer gerührten 1-Gew.-%igen methanolischen Natriumhydroxidlösung zugesetzt, die auf eine Temperatur im Bereich von 50 - 60° C erhitzt worden war. Anschliessend rührte man $1\frac{1}{2}$ Stunden lang bei einer Temperatur von 63° C weiter und dampfte etwa die Hälfte des Methanols ab. Die restliche Lösung wurde durch ein Papierfilter filtriert und in 1,5 1 Wasser einer Temperatur von 25° C unter Rühren eingegossen.

Der gebildete Niederschlag wurde nach 10 Minuten abfiltriert, in 300 ml Methanol suspendiert und erneut abfiltriert. Der Filterrückstand wurde nochmals in 200 ml Methanol suspendiert und in 0,8 1 Wasser einer Temperatur von 25° C eingegossen.

Nach dem erneuten Filtrieren und dem Trocknen unter Vakuum bei einer Temperatur von 60° C erhielt man 1,2 g des Copolymerisates aus Vinylalkohol und Vinylchlorid. Durch das Infrarotspektrum konnte nachgewiesen werden, dass das scharfe Signal der CO-Gruppe der Acetatgruppierung des Ausgangsmateriales bei 1731 $cm^{-1}$ verschwunden war und das verseifte Endprodukt zeigte das breite Signal der -OH Funktion bei 3400 $cm^{-1}$.

Durch die angewandten Reaktionsbedingungen waren also sämtliche Acetatgruppen des Ausgangscopolymerisates zu Hydroxylgruppen verseift worden und das erhaltene Endprodukt war dementsprechend ein Copolymerisat aus 87,0 Mol-% Vinylchlorid plus 13,0 Mol-% Vinylalkohol.

Beispiel 2

Herstellung eines blattförmigen Kunststoff-Formkörpers

Es wurde ein Weichmacher enthaltender Kunststoff-Formkörper hergestellt, indem man 33,5 Gew.-% des nach Beispiel 1 hergestellten Polymermaterials mit 66,5 Gew.-% des Dicarbonsäurediesterweichmachers Bis(1-butyl-pentyl)adipat vermischte und unter Verwendung eines Lösungsmittels zu einem folienförmigen Kunststoff vergoss. Die Herstellung erfolgte nach den Prinzipien des Verfahrens zur Herstellung von ionenselektiven Membranen, das von R. Dohner, D. Wegmann, W.E. Morf und W. Simon in Anal. Chem. 1986, 58, Seiten 2585 - 2589 und der dort zitierten Literatur beschrieben ist. Im vorliegenden Fall enthielt jedoch das so hergestellte folienförmige Material im Gegensatz zu den dort beschriebenen Membranen keine ionenselektive Komponente.

Die erhaltene Kunststoffmembran war weich und besass auf verschiedenen Substraten eine sehr gute Haftfähigkeit.

Beispiel 3

Belegung des Polymermateriales mit einer stabilen Proteinschicht

Die nach dem Verfahren gemäss Beispiel 2 hergestellte Kunststoffmembran wurde montiert und eine Seite derselben wurde in Berührung mit einer wässrigen gepufferten (Tris-Puffer) Rinder-Serumalbuminlösung (7 Gew.-%-ig) in Berührung gebracht. Das verwendete Rinderserumalbumin war ein hoch gereinigtes, von Fettsäuren freies Produkt, das von der Firma Sigma Chemical, USA, bezogen wurde. Nach dieser Behandlung mit dem Rinderserumalbumin wurde die Membran kurz mit destilliertem Wasser gewaschen. Die Belegungsdichte der Membran mit dem Protein wurde bestimmt. Ferner wurde nach dem weiter vorne beschriebenen spektroskopischen Verfahren die Lage der Carbonylgruppen der Proteinmoleküle der Proteinschicht bestimmt.

Anschliessend wurde die mit dem Rinderalbumin vorbehandelte Seite der Membran in 1,0 Gew.-%ige NaCl- Lösung während mehrerer Tage eingetaucht und es wurde immer wieder geschüttelt.

Nach dieser Behandlung zeigte es sich, dass auf der Membran eine unveränderte Belegungsdichte mit der Proteinschicht vorhanden war. Durch die erwähnte spektroskopische Untersuchung konnte ferner gezeigt werden, dass sich durch diese Behandlung die Konformation der Proteinmoleküle der Schicht nicht geändert hatte.

Entsprechende mit dem Rinderserumalbumin vorbehandelte Membranen wurden ferner einer Nachbehandlung mit weiteren Protein enthaltenden Lösungen unterworfen. Es zeigte sich dabei, dass kein Austausch des Serumalbumins gegen Proteine der Lösung erfolgte.

Beispiel 4

Die nach dem Verfahren gemäss Beispiel 3 mit dem Rinderserumalbumin auf einer Seite vorbehandelte Membran wurde mit menschlichem Gesamtblut in Berührung gebracht und während verschiedener Zeiträume im Kühlschrank in Berührung gehalten.

Nach dem Durchspülen der Membranen mit einer 1,0 Gew.-%igen physiologischen Kochsalzlösung war keine Anlagerung von Bestandteilen des menschlichen Gesamtblutes an der Membranoberfläche feststellbar.

**Patentansprüche**

1. Polymermaterial, das hydrophile Substituenten trägt und das nach einem Kontakt mit einem Proteine enthaltenden flüssigen Medium eine stabile Proteinschicht anlagert, dadurch gekennzeichnet, dass das Polymermaterial ein Copolymeres ist, das Monomereinheiten aufweist, die von Alkenen oder Alkinen mit einer oder mehr Mehrfachbindungen stammen, wobei diese Alkene und Alkine gegebenenfalls einen oder mehrere Substituenten tragen, die Chloratome, Bromatome, Arylreste, Carbonsäureestergruppen, Carbonsäureamidgruppen, Nitrilgruppen, veresterte oder verätherte Hydroxylgruppen, Ketogruppen, freie oder acetalisierte Aldehydgruppen sind, und/oder das Copolymerisat Polyesteranteile, Polyurethananteile, Polyamidanteile und/oder Polycarbonatanteile enthält, und wobei 5 - 25 Mol-% der Monomereinheiten des Copolymerisates ein oder mehrere hydrophile Substituenten tragen, die Hydroxylgruppen und/oder Carboxylgruppen sind.

2. Polymermaterial nach Anspruch 1, dadurch gekennzeichnet, dass die Hydroxylgruppen, bzw. Carboxylgruppen entweder direkt an ein Kohlenstoffatom der Hauptkette des Polymermaterials gebunden sind oder an die Hauptkette des Polymermaterials über eine aliphatische, cycloaliphatische oder aromatische Seitenkette mit maximal 6 Kohlenstoffatomen gebunden sind.

3. Polymermaterial nach Anspruch 1 oder 2, dadurch gekennzeichnet, dass es bei Kontakt mit Wasser oder wässrigen Lösungen kaum oder nur geringfügig quillt und dass dabei eine Volumensvergrösserung um höchstens 15%, vorzugsweise höchstens 10%, bezogen auf das Volumen des Polymermaterials vor dem Kontakt mit dem Wasser oder der wässrigen Lösung auftritt.

4. Polymermaterial nach einem der Ansprüche 1 bis 3, dadurch gekennzeichnet, dass es ein Polyvinylhalogenidcopolymerisat oder ein Polyvinylidenhalogenidcopolymerisat ist, in welchem das Halogenid ein Chlorid und/oder Bromid ist, wobei das Chlorid bevorzugt ist und in dem Copolymerisat 5 - 25 Mol-% der Monomereinheiten eine Hydroxylgruppe und/oder Carboxylgruppe als Substituenten tragen und wobei in dem Copolymerisat gegebenenfalls noch zusätzlich Estergruppierungen, Säureamidgruppierungen, Nitrilgruppierungen, Carbonatgruppierungen und/oder Urethangruppierungen vorhanden sind.

5. Polymermaterial nach Anspruch 4, dadurch gekennzeichnet, dass es ein Copolymerisat aus Vinylchlorid und Vinylalkohol ist, in dem 7 - 19 Mol-% der Monomereinheiten, vorzugsweise 11 - 14 Mol-% der Monomereinheiten des Copolymerisates aus Vinylalkohol stammen und wobei in dem Copolymerisat gegebenenfalls noch Monomereinheiten vorhanden sind, die Estergruppen, vorzugsweise veresterte Hydroxylgruppen, als Substituenten tragen.

6. Polymermaterial nach einem der Ansprüche 1 bis 5, dadurch gekennzeichnet, dass es als weitere Komponente einen Weichmacher enthält, wobei dieses Weichmacher enthaltende Copolymerisat vorzugsweise 30 - 50 Gew.-% an dem Copolymerisat und 50 - 70 Gew.-% an dem Weichmacher enthält.

7. Polymermaterial nach Anspruch 6, dadurch gekennzeichnet, dass es als Weichmacher einen Esterweichmacher enthält, vorzugsweise einen Dicarbonsäurediester oder einen Tetracarbonsäuretetraester.

8. Polymermaterial nach einem der Ansprüche 1 bis 7, dadurch gekennzeichnet, dass es in Form von Formkörpern oder Beschichtungen vorliegt, bei deren Verwendung mindestens eine Oberfläche derselben mit einem proteinhaltigen flüssigen Medium oder einem Gewebe von Säugetieren in Berührung kommt.

9. Verwendung des in Form von Formkörpern oder Beschichtungen vorliegenden Polymermateriales gemäss Anspruch 8, dadurch gekennzeichnet, dass man bei der Verwendung mindestens eine Oberfläche des Formkörpers oder der Beschichtung mit einem proteinhaltigen flüssigen Medium oder einem Gewebe von Säugetieren in Berührung bringt, wobei sich dadurch auf dieser Oberfläche des Formkörpers oder der Beschichtung eine Proteinschicht ausbildet, die an dem Polymermaterial so stark verankert ist, dass sie bei einem Kontakt mit einem wässrigen Medium, das gegebenenfalls Proteine und/oder gelöste Salze enthält, nicht von der Polymeroberfläche entfernt und/ oder gegen Fremdproteine ausgetauscht wird.

10. Verwendung nach Anspruch 9, dadurch gekennzeichnet, dass die verwendeten Formkörper oder Beschichtungen Sensoren oder Indikatorvorrichtungen sind, die zur Bestimmung von ionischen oder nicht ionischen Komponenten oder gelösten Gasen in einem wässrigen proteinhaltigen Medium dienen, wobei an der Oberfläche oder den Bereichen nahe der Oberfläche des Sensors oder der Indikatorvorrichtung, die bei der Bestimmung der ionischen, beziehungsweise nichtionischen Komponenten oder der gelösten Gase in dem wässrigen proteinhaltigen Medium mit diesem in Berührung kommt, ein Indikator enthalten ist, der eine Eigenschaft ändert, sobald er mit der zu bestimmenden Komponente, beziehungsweise dem gelösten Gas, in Berührung kommt oder der in einem zweiten Indikator eine derartige Aenderung hervorruft.

11. Verwendung nach Anspruch 10, dadurch gekennzeichnet, dass die verwendeten Formkörper oder Beschichtungen als Indikator eine Substanz enthalten, die bei Anwesenheit der zu bestimmenden Komponente oder des gelösten Gases in dem wässrigen proteinhaltigen Medium ihre Absorption im infraroten, sichtbaren oder ultravioletten Lichtbereich ändert oder diese Substanz das Auftreten oder das Verschwinden einer Fluoreszenz zeigt, oder dass der Indikator ein Biosensor ist, der eine biologisch wirksame Komponente, beispielsweise ein Enzym, ein Antigen, einen Antikörper oder einen Mikroorganismus enthält, wobei bei der Reaktion mit der im wässrigen proteinhaltigen Medium zu bestimmenden Komponente eine Wärmeentwicklung, eine Farbveränderung, eine Niederschlagsbildung, eine Aenderung des pH-Wertes, eine Auflösung eines Niederschlages oder eine solche Veränderung stattfindet, die mit Hilfe eines weiteren Indikators festgestellt werden kann.

12. Verwendung der Polymermaterialien gemäss einem der Ansprüche 1 bis 7, zur Herstellung von Formkörpern oder Beschichtungen, die bei ihrem Gebrauch mit einem Protein enthaltenden flüssigen Medium oder einem Säugergewebe in Berührung kommen, wobei diese Formkörper oder Beschichtungen gegebenenfalls vor ihrer Verwendung mit einem Protein enthaltenden flüssigen Medium in Berührung gebracht werden, wobei dadurch an der Oberfläche der Formkörper oder Beschichtungen eine stabile Proteinschicht angelagert wird, die bei einem nachfolgenden Kontakt dieser Formkörper oder Beschichtungen mit einem Protein enthaltenden wässrigen Medium oder einem Säugergewebe nicht von der Oberfläche des Formkörpers oder der Beschichtung entfernt oder gegen Fremdprotein ausgetauscht wird.

## Claims

1. A polymer material which carries hydrophilic substituents and which, after contact with a protein-containing liquid medium, takes up a stable protein layer, characterized in that the polymer material is a copolymer having monomer units derived from alkenes or alkynes containing one or more multiple bonds, these alkenes and alkynes being unsubstituted or carrying one or more substituents, namely chlorine atoms, bromine atoms, aryl radicals, carboxylic acid ester groups, carboxamide groups, nitrile groups, esterified or etherified hydroxyl groups, keto groups or free or acetalated aldehyde groups, and/or the copolymer contains polyester moieties, polyurethane moieties, polyamide moieties and/or polycarbonate moieties, 5 - 25 mol% of the monomer units of the copolymer carrying one or more hydrophilic substituents, namely hydroxyl groups and/or carboxyl groups.

2. A polymer material according to Claim 1, characterized in that the hydroxyl groups or carboxyl groups are either bonded directly to a carbon atom of the main chain of the polymer material or bonded to the main chain of the polymer material via an aliphatic, cycloaliphatic or aromatic side chain having a maximum of 6 carbon atoms.

3. A polymer material according to Claim 1 or 2, characterized in that it hardly swells at all on contact with water or aqueous solutions, or it swells to only a small extent, and in that there is an associated volume increase of at most 15%, preferably at most 10%, based on the volume of the polymer material prior to contact with the water or aqueous solution.

4. A polymer material according to any one of Claims 1 to 3, characterized in that it is a polyvinyl halide copolymer or a polyvinylidene halide copolymer in which the halide is a chloride and/or bromide, chloride being preferred, and 5 - 25 mol% of the monomer units in the copolymer carry a hydroxyl group and/or carboxyl group as substituents, ester groups, acid amide groups, nitrile groups, carbonate groups and/or urethane groups additionally being present in the copolymer, if appropriate.

5. A polymer material according to Claim 4, characterized in that it is a copolymer of vinyl chloride and vinyl alcohol in which 7 - 19 mol% of the monomer units, preferably 11 - 14 mol% of the monomer units, of the copolymer are derived from vinyl alcohol, monomer units which carry ester groups, preferably esterified hydroxyl groups, as substituents also being present in the copolymer, if appropriate.

6. A polymer material according to any one of Claims 1 to 5, characterized in that it contains a plasticizer as a further component, this plasticized copolymer preferably containing 30 - 50% by weight of the copolymer and 50 - 70% by weight of the plasticizer.

7. A polymer material according to Claim 6, characterized in that it contains an ester plasticizer, preferably a dicarboxylic acid diester of a tetracarboxylic acid tetraester, as the plasticizer.

8. A polymer material according to any one of Claims 1 to 7, characterized in that it is in the form of moulded articles or coatings, at least one surface of which comes into contact with a protein-containing liquid medium or a mammalian tissue during use.

9. Use of the polymer material according to Claim 8, in the form of moulded articles or coatings, characterized in that, during use, at least one surface of the moulded article or coating is brought into contact with a protein-containing liquid medium or a mammalian tissue, resulting in the formation, on this surface of the moulded article or coating, of a protein layer which is so strongly anchored to the polymer material that, on contact with an aqueous medium which may contain proteins and/or dissolved salts, it is not removed from the polymer surface and/or exchanged with foreign proteins.

10. Use according to Claim 9, characterized in that the moulded articles or coatings used are sensors or indicator devices which are used for the determination of ionic or non-ionic components or dissolved gases in an aqueous protein-containing medium, an indicator being contained on that surface or in the regions near that surface of the sensor or indicator device which, in the determination of the ionic or non-ionic components or the dissolved gases in the aqueous protein-containing medium, comes into contact therewith, said indicator changing a property as soon as it comes into contact with the component or dissolved gas to be determined, or causing such a change in a second indicator.

11. Use according to Claim 10, characterized in that the moulded articles or coatings used contain, as an indicator, a substance which, in the presence of the component or dissolved gas to be determined in the aqueous protein-containing medium, changes its absorption in the infrared, visible or ultraviolet light region or shows the appearance or disappearance of a fluorescence, or in that the indicator is a biosensor which contains a biologically active component, for example an enzyme, an antigen, an antibody or a micro-organism, whereby, in the reaction with the component to be determined in the aqueous protein-containing medium, heat is evolved, the colour changes, a precipitate is formed, the pH changes, a precipitate dissolves or a change takes place which can be detected by means of another indicator.

12. Use of the polymer materials according to any one of Claims 1 to 7 for the manufacture of moulded articles or coatings which come into contact with a protein-containing liquid medium or a mammalian tissue during use, these moulded articles or coatings being brought into contact with a protein-containing liquid medium before use, if appropriate, resulting in the uptake, by the surface of the moulded articles or coatings, of a stable protein layer which, on subsequent contact of these moulded articles or coatings with a protein-containing aqueous medium or a mammalian tissue, is not removed from the surface of the moulded article or coating or exchanged with foreign protein.

**Revendications**

1. Matériau polymère comportant des substituants hydrophiles et sur lequel se dépose une couche stable de protéines après un contact avec un milieu liquide contenant des protéines, caractérisé en ce que le matériau polymère est un copolymère présentant des unités monomères qui proviennent des alcènes ou des alcynes avec une ou plusieurs liaisons multiples, lesdits alcènes et alcynes comportant éventuellement un ou plusieurs substituants qui sont des atomes de chlore, des atomes de brome, des radicaux aryle, des groupes d'esters d'acides carboxyliques, des groupes d'amides d'acides carboxyliques, des groupes nitrile, des groupes hydroxyle estérifiés ou éthérifiés, des groupes cétone, des groupes aldéhydes libres ou acétalisés et/ou le copolymère contient des fractions de polyester, des fractions de polyuréthane, des fractions de polyamides et/ou des fractions de polycarbonates, tandis que 5 à 25 moles % des unités monomères du copolymère portent un ou plusieurs substituants hydrophiles qui sont des groupes hydroxyle et/ou carboxyle.

2. Matériau polymère Selon la revendication 1, caractérisé en ce que les groupes hydroxyle ou les groupes carboxyle sont liés soit directement à un atome de carbone de la chaîne principale du matériau polymère, soit à la chaîne principale du matériau polymère par l'intermédiaire d'une chaîne latérale aliphatique, cycloaliphatique ou aromatique comportant au maximum 6 atomes de carbone.

3. Matériau polymère selon la revendication 1 ou 2, caractérisé en ce que celui-ci gonfle à peine en cas de contact avec de l'eau ou des solutions aqueuses ou ne gonfle que légèrement et que l'augmentation de volume résultante atteint au maximum 15% et, de préférence, au maximum 10% par rapport au volume du matériau polymère avant le contact avec l'eau ou la solution aqueuse.

4. Matériau polymère selon l'une des revendications 1 à 3, caractérisé en ce que c'est un copolymère d'halogénure de polyvinyle ou de polyvinylidène dans lequel l'halogénure est un chlorure et/ou un bromure, tandis que le chlorure est préféré et que, dans le copolymère, 5 à 25% molaires des unités monomères portent un groupe hydroxyle et/ou un groupe carboxyle comme substituant et qu'il existe éventuellement aussi dans le copolymère des groupes ester, des groupes d'amidés d'acides, des groupes nitrile, des groupes carbonate et/ou des groupes uréthane.

5. Matériau polymère selon la revendication 4, caractérisé en ce que c'est un copolymère de chlorure de vinyle et d'alcool vinylique dans lequel 7 à 19% molaires des unités monomères et, de préférence, 11 à 14% molaires des unités monomères du copolymère proviennent de l'alcool vinylique, tandis qu'il existe éventuellement aussi dans le copolymère des unités monomères qui portent des groupes ester et, de préférence, des groupes hydroxyle estérifiés comme substituants.

6. Matériau polymère Selon l'une des revendications 1 à 5, caractérisé en ce qu'il contient un plastifiant comme autre constituant, tandis que ce copolymère contenant un plastifiant contient de préférence 30 à 50% en poids de copolymère et 50 à 70% en poids de plastifiant.

7. Matériau polymère selon la revendication 6, caractérisé en ce qu'il contient comme plastifiant un plastifiant à base d'ester et, de préférence, un diester d'acide dicarboxylique ou un tétraester d'acide tétracarboxylique.

8. Matériau polymère selon l'une des revendications 1 à 7, caractérisé en ce qu'il se présente sous forme de pièces de forme ou de revêtements dont au moins une surface vient au contact d'un milieu liquide contenant des protéines ou d'un tissu de mammifère au cours de leur utilisation.

9. Utilisation du matériau polymère se présentant sous forme de pièce de forme ou de revêtement selon la revendication 8, caractérisé en ce qu'au cours de l'utilisation, au moins une surface de la pièce de forme ou du revêtement est mise en contact avec un milieu liquide contenant des protéines ou avec un tissu de mammifère, tandis qu'il se forme ainsi sur cette surface de là pièce de forme ou du revêtement une couche de protéines qui est si fortement ancrée au matériau polymère qu'en cas de contact avec un milieu aqueux qui contient éventuellement des protéines et/ou des sels dissous, elle n'est pas éliminée de la surface du polymère et/ou n'est pas échangée par des protéines étrangères.

10. Utilisation, selon la revendication 9, caractérisée en ce que les pièces de forme ou les revêtements utilisés sont des détecteurs ou des dispositifs indicateurs destinés à la détermination des constituants ioniques ou non ioniques ou des gaz dissous, la surface ou la zone proche de la surface du détecteur ou dispositif indicateur, venant en contact du milieu protéinique aqueux lors de la détermination des constituants respectivement ioniques, non-ioniques ou des gaz dissous dans ce milieu, étant pourvue d'un indicateur dont une propriété se modifie dès qu'il vient en contact avec les constituants à déterminer ou avec le gaz dissous ou qui provoque une modification de ce genre dans un second indicateur.

11. Utilisation, selon la revendication 10, caractérisée en ce que les pièces de forme ou les revêtements contiennent comme indicateur une substance qui, en présence des constituants à déterminer ou du gaz dissous dans le milieu aqueux contenant des protéines, modifie son absorption dans le domaine de la lumière infrarouge, visible ou ultraviolette ou que cette substance manifeste l'apparition ou la disparition d'une fluorescence ou bien que l'indicateur est un biodétecteur qui contient un constituant biologiquement actif, comme, par exemple, un enzyme, un antigène, un anticorps ou un micro-organisme, en sorte qu'il se produit au cours de la réaction avec les constituants à déterminer contenus dans le milieu aqueux contenant des protéines un dégagement de chaleur, une modification de teinte, la formation d'un précipité, une modification de la valeur du pH, la dissolution d'un précipité ou toute autre modification qui peut être constatée à l'aide d'un autre indicateur.

12. Utilisation des matériaux polymères selon l'une des revendications 1 à 7 pour la préparation des pièces de forme ou de revêtements qui viennent en contact avec un milieu liquide contenant une protéine ou avec un tissu de mammifères au cours de leur utilisation, tandis que ces pièces de forme ou ces revêtements sont mis éventuellement en contact, avant leur utilisation, avec un milieu liquide contenant une protéine, ce qui provoque le dépôt à la surface des pièces de forme ou des revêtements d'une couche stable de protéines qui n'est pas éliminée de la surface des pièces de forme Ou des revêtements ou qui n'est pas échangée par des protéines étrangères au cours d'un contact ultérieur de ces pièces de forme ou de ces revêtements avec un milieu aqueux contenant des protéines ou avec un tissu de mammifère .